# EUROPEAN PATENT APPLICATION

(11) **EP 2 623 151 A1**
(43) Date of publication of application: **07.08.2013**
(21) Application number: 11828572.5
(22) Date of filing: 11.07.2011
(51) Int. Cl.: A61M 25/08, A61F 2/84

(54) **INTRODUCER SHEATH, PLACEMENT DEVICE FOR BLOOD VESSEL TREATMENT INSTRUMENT, AND METHOD FOR SHORTENING INTRODUCER SHEATH**

(30) Priority: 28.09.2010 JP 2010217881
(71) Applicant: Terumo Kabushiki Kaisha, Tokyo 151-0072 (JP)
(72) Inventor: SAWADA Akira, Fujinomiya-shi Shizuoka 418-0015 (JP)
(74) Representative: Dossmann, Gérard
(86) International application number: PCT/JP2011/065757
(87) International publication number: WO 2012/043011

(57) **Abstract**

A placement device (10) for a blood vessel treatment instrument has an introducer sheath (14) functioning as an outer tube, and also has an inner tube (16). The introducer sheath (14) has a flexible tube-shaped sheath body (18) and a hub (20) into which the base end of the sheath body (18) is inserted. The hub (20) takes up the base end of the sheath body (18) into the hub (20) by means of take-up shafts (30, 32) while tearing the base end of the sheath body (18) by cutting blades (72, 74), and thus the length of extension of the sheath body (18) from the hub (20) can be shortened.

## Description

### [Technical Field]

The present invention relates to an introducer sheath of which the overall length can be shortened, a placement device for a blood vessel treatment instrument, and a method for shortening an introducer sheath.

### [Background Art]

At the time of inserting a catheter into a blood vessel for diagnosis or treatment, in general, an introducer sheath is used as an access route which interconnects the exterior of the living body and the inside of the blood vessel. A primary purpose of this is to reduce the damage to a blood vessel which damage is generated, at the time of sequentially using a plurality of catheters, due to the process in which the catheters different in diameter are inserted into and withdrawn from a blood vessel puncture part a plurality of times. A secondary purpose is to ensure easy insertion at the time of inserting a catheter having a non-smooth surface, such as a large-diameter balloon catheter or a balloon-expandable stent, and to avoid damage to a blood vessel.

On the other hand, in the case of a stent graft system used for treatment of aortic aneurysm (hereinafter referred also to simply as "system"), an introducer sheath is rarely used and the system is often inserted directly into a blood vessel. The stent graft system is a treatment device used in a stent graft insertion procedure in which a stent graft is carried to a treatment site where the aneurysm is present, then the stent graft is allowed to expand by its self-expanding force and is placed indwelling in situ. The stent graft system is by far larger than common catheters in shaft outside diameter, and this is why the introducer sheath is rarely used therewith.

A general stent graft system has an outer tube for housing a stent graft on the inner circumference of a distal portion thereof, and an inner tube slidably inserted inside the outer tube. The general stent graft system is so configured that with the outer tube slid proximally relative to the inner tube, the stent graft is allowed to expand radially by its self-expanding force, to be placed indwelling in a blood vessel (refer to, for example, Japanese Patent Laid-open No. 2005-270395).

Most of the products of stent graft are of self-expanding type. As for the outer surface of the stent graft's shaft section to be inserted into the living body, in many cases, the stent part is also housed in a smooth tube, like in the cases of other self-expandable stents. Therefore, non-use of an introducer sheath at the time of inserting a stent graft system into a blood vessel would not produce the problem of damage to the blood vessel.

In the stent graft insertion procedure, however, there are cases where after the placement of a stent graft, another stent graft is added as an extension graft. Besides, there are cases where a balloon catheter is inserted for the purpose of adhering the stent graft to the blood vessel more firmly. Therefore, there still remains the problem of damage to the blood vessel due to the repetition of insertion and withdrawal.

In order to cope with such a problem, there is a method in which the outer tube of the system is employed as an introducer sheath for other treatment device(s). In this method, after the first stent graft is inserted and placed indwelling, the outer tube (and the guide wire) of the system is left in the blood vessel, whereas the other component parts (inner tube, etc.) constituting the system are all pulled out. Then, the outer tube thus left in the blood vessel is used as an introducer sheath at the time of inserting other treatment device(s). This prevents spreading of the damage to the blood vessel puncture part at the time of inserting additional stent graft(s) or balloon catheter(s).

### [Summary]

In most of the stent graft systems according to the related art as above-described, it is necessary to insert the outer tube to the position (depth) where the stent graft is to be actually placed indwelling. Therefore, the overall length of the system inclusive of the outer tube is greater, as compared with common introducer sheaths. Therefore, in the case of using the outer tube of the system as it is as an introducer sheath at the time of inserting other treatment device, the treatment device must have an overall length not less than the overall length of the outer tube. Thus, treatment devices which are small in overall length cannot be used. In addition, a treatment device with an excessively large length is difficult to manipulate.

The present invention has been made in consideration of the above-mentioned circumstances. Accordingly, it is an object of the present invention to provide an introducer sheath which can be used as an introducer sheath for a short treatment device and which eliminates the need to use an excessively long treatment device, and also to provide a placement device for a blood vessel treatment instrument, and a method for shortening an introducer sheath.

In order to attain the above object, according to the present invention, there is provided an introducer sheath into which a long shaft is inserted, including: a flexible tube-shaped sheath body; and a hub into which a proximal portion of the sheath body is inserted, characterized in that the hub takes up a proximal portion of the sheath body into the hub while tearing the proximal portion of the sheath body, whereby the length of extension of the sheath body from the hub can be shortened.

According to the just-mentioned configuration, it is possible, after the introducer sheath is employed as an outer tube of a blood vessel treatment instrument placement device to be used for placing a blood vessel treatment instrument (stent graft), to shorten the overall length of the sheath body of the introducer sheath. Therefore, a treatment device shorter than the overall length of the original introducer sheath can be inserted into a blood vessel by utilizing the introducer sheath. In addition, it is unnecessary to use an excessively long treatment device.

In the above-mentioned introducer sheath, preferably, the hub has: a cutting section by which slits along an axial direction are formed in the sheath body; and a plurality of take-up shafts for respectively taking up terminal pieces of the sheath body torn by the slits.

According to the just-mentioned configuration, it is possible, by taking up the terminal portions of the sheath body by the plurality of take-up shafts, to draw the sheath body into the hub and simultaneously to form slits in the sheath body by the cutting section. Therefore, take-up of the sheath body and formation of the slits are concurrently carried out by rotating the take-up shafts, so that the effective length of the sheath body can be easily shortened by a single operation (rotating operation).

In the above-mentioned introducer sheath, preferably, the cutting section forms the slits in portions of the sheath body which are on opposite sides with respect to a circumferential direction, and the plurality of take-up shafts are two take-up shafts disposed at positions spaced from each other along the direction of splitting of the sheath body by the slits.

According to the just-mentioned configuration, the proximal portion of the sheath body is torn up into two by the cutting section, and the resulting terminal ends are taken up by the two take-up shafts spaced from each other in the direction of splitting of the sheath body. Therefore, the sheath body can be smoothly taken up with a small number of component parts.

In the above-mentioned introducer sheath, preferably, the cutting section has a plurality of cutting blades by which the slits are formed in circumferential-directionally different portions of the sheath body.

According to the just-mentioned configuration, sharp cutting blades are used as the cutting section, whereby the cutting resistance at the time of drawing the sheath body into the hub can be reduced. This ensures that the torque required at the time of taking up the terminal portions of the sheath body can be reduced, and the taking-up operation can be carried out more easily.

In the above-mentioned introducer sheath, preferably, the hub further has an interlocking mechanism by which the plurality of take-up shafts are rotated in an interlocked manner.

According to the just-mentioned configuration, all the take-up shafts are rotated simultaneously, so that the terminal portions torn apart can be taken up in a well-balanced manner.

In the above-mentioned introducer sheath, preferably, the hub has rotation restraining mechanisms for inhibiting the take-up shafts from rotating in an unwinding direction.

According to the just-mentioned configuration, the take-up shafts would not be rotated in the direction for unwinding the terminal portions of the sheath body having once been taken up, so that the sheath body is inhibited from moving in the extending direction relative to the hub. Therefore, the slits formed by the cutting section would not be exposed to the outside of the hub, so that blood can be prevented from leaking to the exterior of the hub through the slits.

In the above-mentioned introducer sheath, preferably, the hub has: a hub body provided with a hollow section in which the take-up shafts and the proximal portion of the sheath body are housed; and a rotational operating section which is rotationally operated from outside of the hub body to thereby rotate the take-up shafts, the hub body being configured to be liquid-tight so that a liquid flowing into the inside of the hub through the sheath body does not leak to the exterior.

According to the just-mentioned configuration, it is ensured that even when blood flows into the hub through the sheath body, the blood is prevented from leaking to the exterior of the hub.

In addition, according to the present invention, there is provided a blood vessel treatment instrument placement device by which a blood vessel treatment instrument having a self-expanding function is fed to and placed indwelling in a desired treatment site in a blood vessel, characterized in that the placement device includes an introducer sheath having a sheath body for housing the blood vessel treatment instrument on an inner circumference of a distal portion thereof, and an inner tube slidably inserted inside the sheath body; the introducer sheath has the sheath body which is flexible and tube-like in shape, and a hub in which a proximal portion of the sheath body is inserted; and the hub takes up a proximal portion of the sheath body into the hub while tearing the proximal portion of the sheath body, whereby the length of extension of the sheath body from the hub can be shortened.

According to the just-mentioned configuration, after the placement of the blood vessel treatment instrument, it is possible, by pulling the inner tube out of the sheath body and shortening the effective length of the sheath body, to employ the introducer sheath as an introducer sheath for a treatment device which is shorter than the overall length of the original introducer sheath. In addition, it is unnecessary to use an excessively long treatment device.

Besides, according to the present invention, there is provided a method for shortening an introducer sheath in which a long shaft is inserted, the method including: tearing a proximal portion of a sheath body; drawing the sheath body into the hub in which the proximal portion of the sheath body is inserted; and taking up terminal pieces of the sheath body having been torn.

### [Brief Description of Drawings]

[FIG. 1]

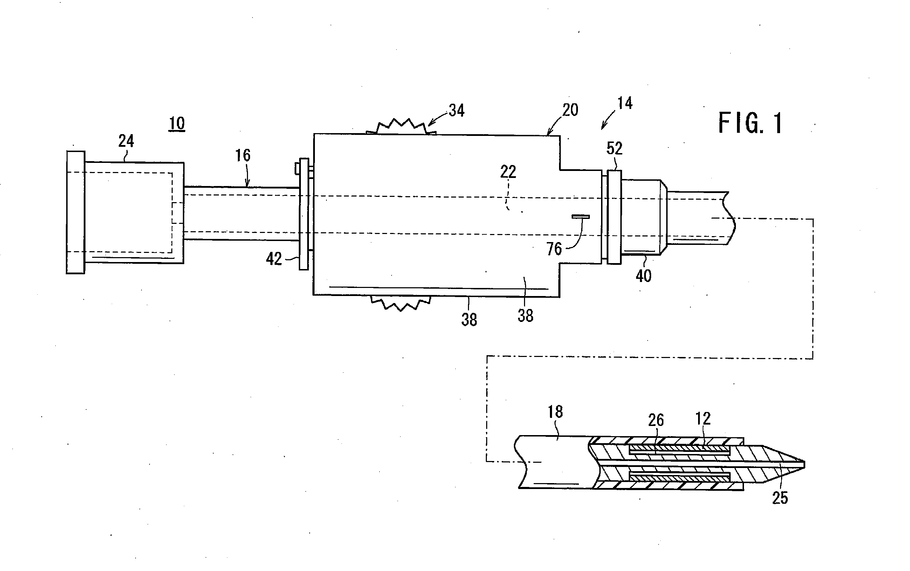

   FIG. 1 is a partly omitted side view of a blood vessel treatment instrument placement device according to an embodiment of the present invention.
[FIG. 2]

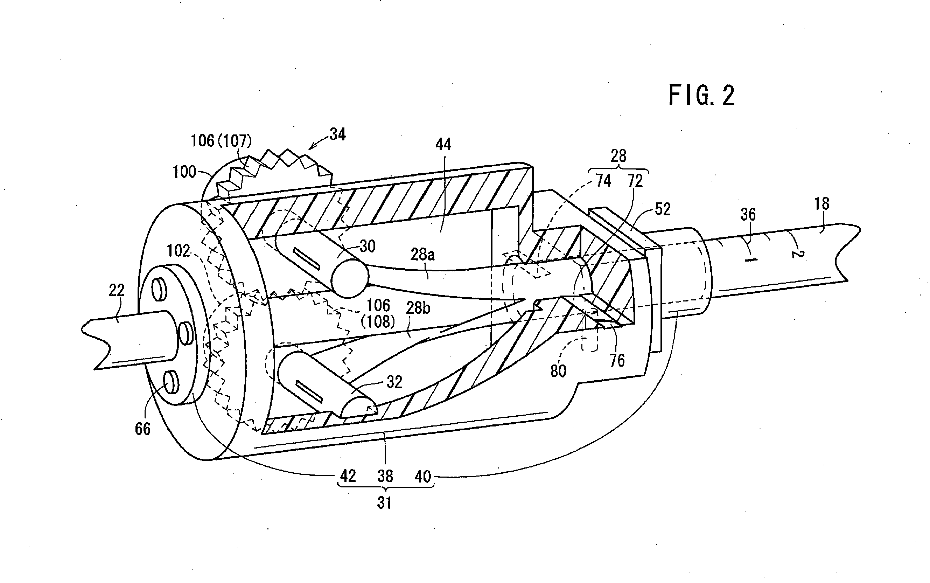

   FIG. 2 is a partly omitted perspective view of an introducer sheath shown in FIG. 1.
[FIG. 3]

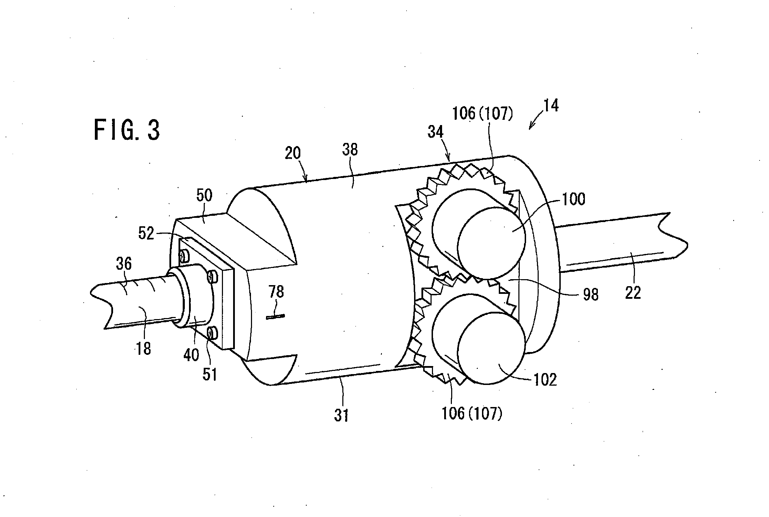

   FIG. 3 is a partly omitted perspective view of the introducer sheath shown in FIG. 1.
[FIG. 4]

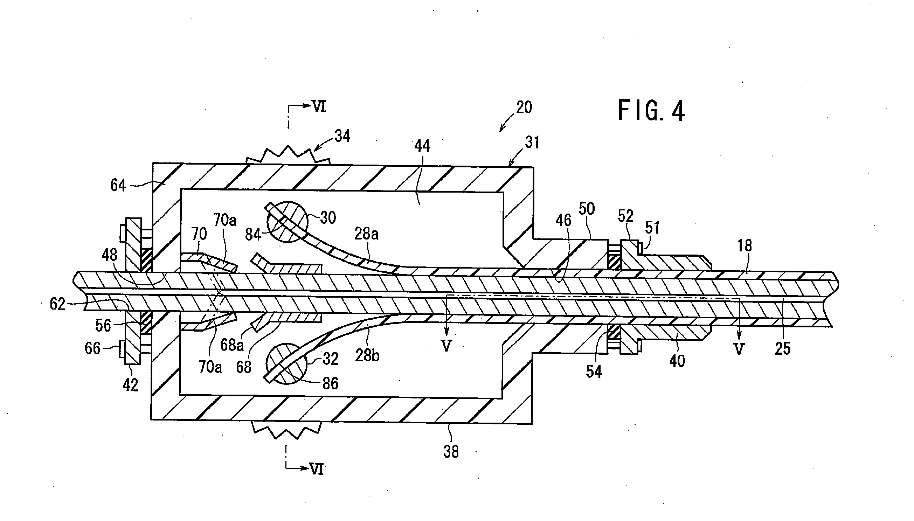

   FIG. 4 is a partly omitted schematic longitudinal sectional view of the introducer sheath shown in FIG. 1.
[FIG. 5]

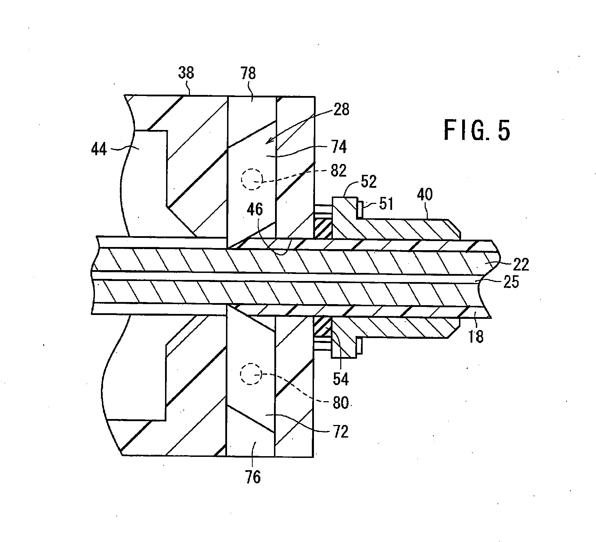

   FIG. 5 is a sectional view taken along line V-V of FIG. 4.
[FIG. 6]

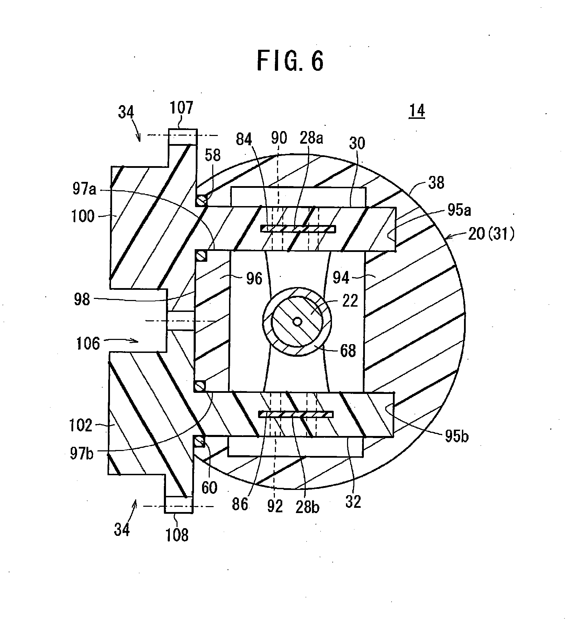

   FIG. 6 is a sectional view taken along line VI-VI of FIG. 4.
[FIG. 7]

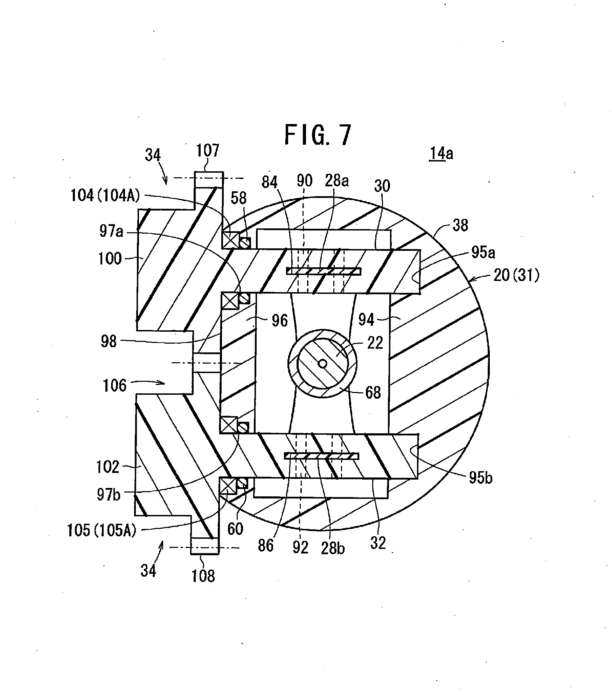

   FIG. 7 is a transverse sectional view of an introducer sheath according to a modification.
[FIG. 8]

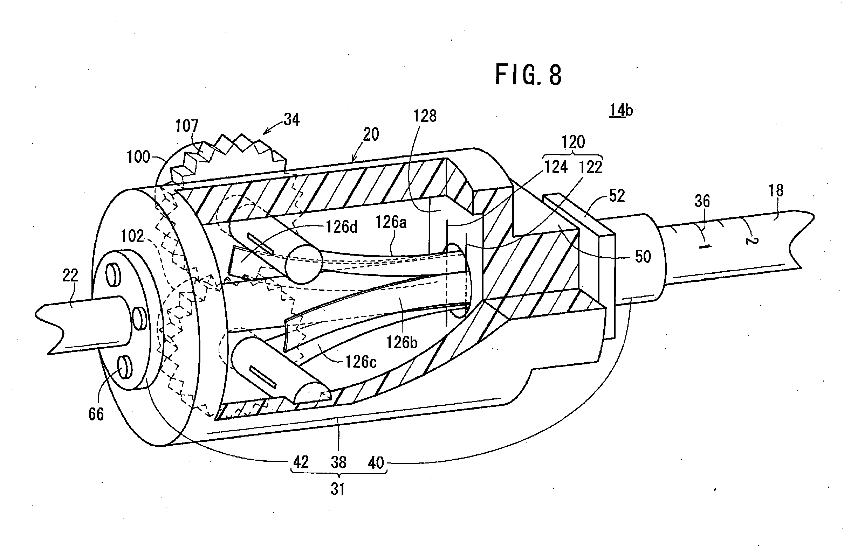

   FIG. 8 is a partly omitted schematic perspective view of an introducer sheath according to another modification.

### [Description of Embodiments]

Now, an introducer sheath, a blood vessel treatment instrument placement device, and a method for shortening an introducer sheath according to the present invention will be described below, while showing preferred embodiments and referring to the accompanying drawings. For convenience of description, the right side in FIGS. 1, 4 and 5 will be referred to as "distal end," the left side in the FIGS. 1, 4 and 5 as "proximal end (rear end)," the right upper side in FIG. 2 as "distal end," the left lower side in FIG. 2 as "proximal end (rear end)," the left lower side in FIG. 3 as "distal end," and the right upper side in FIG. 3 as "proximal end (rear end)."

The blood vessel treatment instrument placement device 10 (hereinafter referred also to simply as "placement device 10") shown in FIG. 1 is a device by which a stent graft 12 as a blood vessel treatment instrument having a self-expanding function is fed to and placed indwelling at a desired treatment site in a blood vessel. The stent graft 12 includes a stent formed in a tubular shape by knitting or braiding metallic wires, and a graft composed of a tubular cover disposed on the outer circumference of the stent. On one hand, the stent graft 12 can be contracted in diameter through elastic deformation by a radially inward force; on the other hand, the stent graft 12 has a self-expanding force for restoring its original size by an elastic force upon removal of the radially inward force. Thus, the stent graft 12 is configured as a self-expanding stent graft.

As shown in FIG. 1, the placement device 10 has an introducer sheath 14 and an inner tube 16. In performing a stent graft insertion procedure, the placement device 10 is used with the inner tube 16 inserted in the introducer sheath 14.

The introducer sheath 14 has a sheath body 18 and a hub 20. The sheath body 18 functions as an outer tube which houses the stent graft 12 on an inner circumference of a distal portion thereof. The sheath body 18 is formed from a flexible (elastic) material, and has a lumen having a substantially constant inside diameter along the axial direction thereof. Examples of the material of the sheath body 18 include bio-compatible synthetic resins selected from among polyamides, polyethylene, fluoro-resins, polyimides and the like.

The hub 20 is a hollow member provided at (connected to) a proximal portion of the sheath body 18, and the hollow part is communicating with the lumen of the sheath body 18. Incidentally, detailed configuration of the hub 20 will be described later.

The inner tube 16 is inserted in the introducer sheath 14 in an axially slidable manner, and includes an inner tube body 22 and a hub 24. The inner tube body 22 is a flexible pipe-shaped body longer than the sheath body 18, and is provided therein along its axial direction with a guide wire lumen in which a guide wire is inserted. The inner tube body 22 has a maximum outside diameter not greater than the inside diameter of the sheath body 18 of the introducer sheath 14. Therefore, the inner tube body 22 can be pulled out of the sheath body 18 and the hub 20.

The inner tube body 22 is formed, in an outer circumferential portion near a distal portion thereof, with an annular groove 26 functioning as a housing section in which to house the stent graft 12. In FIG. 1, the stent graft 12 is covered by the sheath body 18 and, hence, inhibited from expansion. Examples of the material of the inner tube 16 include bio-compatible synthetic resins selected from among polyamides, polyethylene, fluoro-resins, polyimides and the like. The hub 20 is a hollow member provided at (connected to) a proximal portion of the inner tube 16.

In performing a stent graft insertion procedure by use of the placement device 10 configured as above, the placement device 10 in the state as shown in FIG. 1 is gradually inserted into a blood vessel along a guide wire precedently inserted in the blood vessel, to bring a distal portion of the placement device 10 to a desired treatment site. Upon the arrival of the distal portion at the desired treatment site, the introducer sheath 14 is moved (slid) in the proximal direction relative to the inner tube 16, so as to shift a distal portion of the sheath body 18 to the proximal side of the stent graft 12. This results in that the stent graft 12, having been restrained by the inner circumferential surface of the sheath body 18 from expansion, expands to be pressed against the blood vessel under its self-expanding force. Consequently, the stent graft 12 is fixed and placed indwelling in the blood vessel.

The introducer sheath 14 is so configured that the inner tube 16 can be pulled out. Further, in order that the introducer sheath 14 is applicable also to short other treatment devices (for example, a balloon catheter), it is so configured that the effective length of the sheath body 18 (the length of extension of the sheath body 18 from the hub 20) can be shortened. In FIGS. 2 to 4, the sheath body 18 is in an initial state in which its effective length is not shortened.

As shown in FIG. 2, a proximal portion of the sheath body 18 is inserted into and fixed in the hub 20. The proximal portion of the sheath body 18 is torn up into plural (in the example shown, two) pieces, and the terminal pieces 28a and 28b are each held inside the hub 20. The hub 20 holding the proximal portion of the sheath body 18 in such a state takes up the proximal portion of the sheath body 18 thereinto while tearing the proximal portion. The hub 20 includes a hub body 31, a cutting section 28, a plurality of take-up shafts 30 and 32, and an operating section 34.

In order that how much the effective length of the sheath body 18 has been shortened can be seen, graduations 36 with numerals are provided on an outer circumferential surface of a proximal portion of the sheath body 18. The graduations, in cooperation with a distal member 40 which will be described later, indicate the length by which the sheath body 18 has been shortened. This point will be described later.

The hub body 31 includes a trunk member 38, the distal member 40, and a proximal member 42. The trunk member 38 has a hollow structure. As shown in FIG. 4, the trunk member 38 is provided with a hollow section 44, a distal-side opening 46, and a proximal-side opening 48, and is further provided with a distal component section 50 on the distal side relative to the hollow section 44. Inside the hollow section 44, the proximal portion of the sheath body 18 is located, and the take-up shafts 30 and 32 are disposed.

The distal-side opening 46 is a cylindrical hole which extends toward the distal side from the hollow section 44 and is approximately equal in diameter to the outer shape of the sheath body 18. The proximal side of the sheath body 18 is inserted through the distal-side opening 46. The proximal-side opening 48 is a cylindrical hole which extends toward the proximal side from the hollow section 44 and is approximately equal in diameter to the outer shape of the inner tube 16. The distal-side opening 46 and the proximal-side opening 48 are coaxial with each other, for permitting the inner tube 16 to be inserted therethrough.

The distal member 40 is a hollow cylindrical member having an inside diameter approximately equal to the diameter of the outer shape of the sheath body 18, and has a flange section 52 on the proximal side. The flange section 52 is fixed to the distal component section 50 by a fixing part or parts 51 such as a screw or screws.

The distal end face of the distal member 40 has a function of indicating the length by which the sheath body 18 has been shortened, in cooperation with the graduations 36 imparted to the outer circumferential surface of the sheath body 18. Specifically, when the sheath body 18 is in the initial state, a zero position of the graduations 36 (the graduation at the most proximal side) coincides with the distal end face of the distal member 40.

Between the flange section 52 of the distal member 40 and the distal component section 50, there is disposed a ring-shaped seal member 54 formed of an elastic material. An inner circumferential surface of the seal member 54 and an outer circumferential surface of the sheath body 18 are in secure contact with each other, over the whole circumference. This ensures liquid-tight sealing between the inner circumferential surface of the hub body 31 and the outer circumferential surface of the sheath body 18, at a distal-side portion of the hub body 31.

The material constituting the seal member 54 is not particularly restricted. Examples of the material which can be used include various rubbers such as natural rubber, butyl rubber, isoprene rubber, butadiene rubber, styrene-butadiene rubber, and silicone rubber. Incidentally, for seal members 56, 58 and 60 which will be described later, also, the materials mentioned above as material for the seal member 54 can be used.

The proximal member 42 is a circular disk-shaped member having an opening 62 approximately equal in diameter to the outer shape of the inner tube 16. The proximal member 42 is fixed to a rear wall 64 of the trunk member 38 by a fixing part or parts 66 such as a screw or screws. Between the proximal member 42 and the rear wall 64 is disposed a ring-shaped seal member 56 which is formed from an elastic material. An inner circumferential surface of the seal member 56 and an outer circumferential surface of the inner tube 16 are in secure contact with each other, over the whole circumference. This ensures liquid-tight sealing between the inner circumferential surface of the hub body 31 and the outer circumferential surface of the inner tube 16, at a proximal-side portion of the hub 31.

In order that another treatment device is smoothly guided to the distal-side opening 46 at the time of inserting the treatment device from the proximal side, the trunk member 38 is provided with a tubular guide member 68 in the hollow section 44 thereof. A proximal portion 68a of this guide member 68 is formed in a flare shape spreading toward the proximal side so that the distal end of the treatment device is smoothly inserted therethrough. Incidentally, the guide member 68 is omitted in FIG. 2.

When the inner tube 16 is pulled out of the introducer sheath 14 in the condition where the introducer sheath 14 is inserted in a blood vessel, blood flows into the hollow section 44 through the sheath body 18. In view of this, as shown in FIG. 4, a check valve 70 as a valve element for hemostasis is provided on the front side of the proximal-side opening 48. The check valve 70 has a plurality of valve elements 70a divided in the circumferential direction. Incidentally, the check valve 70 is omitted in FIG. 2.

In the condition where the inner tube body 22 is inserted, as indicated by solid lines in FIG. 4, the check valve 70 is in a valve-opened state in which the valve elements 70a are spread radially outward. In the condition where the inner tube body 22 is pulled out, as indicated by imaginary lines in FIG. 4, the check valve 70 is in a valve-closed state in which the valve elements 70a are displaced radially inward to be closed. The check valve 70 ensures that even when the inner tube 16 is pulled out of the introducer sheath 14 inserted in a blood vessel, blood can be prevented from leaking to the outside of the hub 20.

Materials for forming the trunk member 38, the distal member 40 and the proximal member 42 are not particularly limited. For example, various metallic materials and various plastics and the like can be used singly or in combination.

As shown in FIGS. 2 and 5, the cutting section 28 is disposed in the distal component section 50 constituting a portion on the distal side of the hollow section 44, in the trunk member 38. The cut section 28 is a section by which slits along the axial direction are formed in the sheath body 18. The cutting section 28 in the example shown is so configured as to form slits in portions of the sheath body 18 which are located circumferentially opposite to each other, namely, at portions deviated from each other by 180 degrees in circumferential phase. Specifically, the cutting section 28 has plural (in the example shown, two) cutting blades 72 and 74.

The two cutting blades 72 and 74 are disposed at positions between which the sheath body 18 interposed, with their cutting edges oriented toward the side of the sheath body 18. By the cutting edges, slits along the axial direction are formed in the sheath body 18. The material constituting the cutting blades 72 and 74 is not specifically restricted; for example, various metallic materials (stainless steel, etc.) can be used. With the cutting blades 72 and 74 formed from a metallic material, it is easy to form the cutting edges.

As shown in FIG. 5, the trunk member 38 (the distal component section 50) is formed with plural (two) through-holes 76 and 78, each of which has its inner end opened at the inner circumferential surface of the distal-side opening 46 and has its outer end opened at the outer surface of the trunk member 38. The through-holes 76 and 78 function as slots in which the cutting blades 72 and 74 are accommodated (disposed). The cutting blades 72 and 74 are inserted from the outer ends of the through-holes 76 and 78, and are positioned and fixed in the through-holes 76 and 78 by fixing pins 80 and 82.

The cutting edges of the cutting blades 72 and 74 protrude from the inner ends of the through-holes 76 and 78 (namely, from the inner surface of the distal-side opening 46) by an amount corresponding roughly to the material thickness (pipe wall thickness) of the sheath body 18, and is not making contact with the inner tube body 22. When the sheath body 18 is moved proximally, therefore, the sheath body 18 is cut by the cutting blades 72 and 74 along the axial direction, but the inner tube body 22 is not formed with any cut.

Thus, the cutting blades 72 and 74 are positioned by being inserted into and fixed in the through-holes 76 and 78; therefore, the amounts of protrusion of the cutting edges from the inner circumferential surface of the distal-side opening 46 can be accurately set, and this state can be securely maintained. Incidentally, while each of the through-holes 76 and 78 in the example shown extends in a direction orthogonal to the axis of the distal-side opening 46, it may extend in a direction inclined relative to the axis.

The configuration for disposing and fixing the cutting blades 72 and 74 in the trunk member 38 is not restricted to the one shown in FIG. 5. For example, a configuration may be adopted wherein the hollow section 44 is extended toward the distal side, and a structure for disposing and fixing the cutting blades 72 and 74 is provided in the extended part.

As shown in FIGS. 2 and 4, the terminal pieces 28a and 28b of the sheath body 18 having been torn are fixed respectively to the plural (two) take-up shafts 30 and 32. The take-up shafts 30 and 32 are members for respectively taking up the terminal pieces 28a and 28b of the sheath body 18 having been torn. The take-up shafts 30 and 32 are disposed on the proximal side of the cutting section 28 in the hub body 31, and are rotatably supported on the trunk member 38.

The two take-up shafts 30 and 32 in the example shown are arranged at positions spaced from each other along the direction of splitting (the vertical direction in FIGS. 2 and 4) of the sheath body 18 by the slits. In addition, the two take-up shafts 30 and 32 are located on opposite sides with reference to the axis of the hub 20 (the axis of the distal-side opening 46 and the proximal-side opening 48). Furthermore, the axes (rotational center lines) of the two take-up shafts 30 and 32 are parallel to each other.

As shown in FIG. 6, the take-up shafts 30 and 32 are provided with holes 84 and 86 extending along the axial direction and penetrating in a diametral direction. The terminal pieces 28a and 28b of the sheath body 18 are inserted into the holes 84 and 86, and are fixed in situ by a plurality of fixing pins 90 and 92 arranged along the axial direction of the take-up shafts 30 and 32. Incidentally, the fixation of the terminal pieces 28a and 28b of the sheath body 18 to the take-up shafts 30 and 32 may be performed by other method such as adhesion or fusion bonding.

As shown in FIG. 6, one-side end portions of the take-up shafts 30 and 32 are inserted in and supported by bottomed cylindrical holes 95a and 95b provided in a one-side wall 94 of the trunk member 38 of the hub body 31. Other-side end portions of the take-up shafts 30 and 32 penetrate holes 97a and 97b provided in another-side wall 96 of the trunk member 38. Between the take-up shafts 30 and 32 and the holes 97a and 97b are disposed seal members 58 and 60 formed of an elastic material, whereby liquid-tight sealing is made between the take-up shafts 30 and 32 and the holes 97a and 97b.

In addition, at the other-side end portions of the take-up shafts 30 and 32, an operating section 34 to be operated to rotate the take-up shafts 30 and 32 is provided outside of the hub body 31. In the configuration shown, the operating section 34 includes two operating wheels 100 and 102 which can be rotationally operated. As shown in FIGS. 3 and 6, the hub body 31 is formed in a side portion thereof with a recess 98 having a flat bottom, and the operating wheels 100 and 102 are disposed in the recess 98.

In this embodiment, the hub 20 further has an interlocking mechanism 106 by which rotations of the take-up shafts 30 and 32 are interlocked with each other. In the configuration shown, the interlocking mechanism 106 includes intermeshing gears 107 and 108 which are provided at outer circumferential portions of the operating wheels 100 and 102. This ensures that when one of the two operating wheels 100 and 102 is rotated, the other is rotated in the reverse direction.

The introducer sheath 14 configured as above-described can, after a stent graft insertion procedure, be used as an introducer sheath for other treatment device (for example, a balloon catheter) by a method in which the inner tube 16 is pulled off on the proximal side and then the effective length of the sheath body 18 is shortened.

In order to shorten the effective length of the sheath body 18, it is necessary only to rotate the operating section 34. Specifically, when the operating wheels 100 and 102 are rotated, the take-up shafts 30 and 32 connected to the operating wheels 100 and 102 are rotated and the terminal pieces 28a and 28b are taken up by the take-up shafts 30 and 32, simultaneously when the slits are formed by the two cutting blades 72 and 74. As a result, the sheath body 18 is drawn into the hub 20, and the effective length is shortened. The amount by which the sheath body 18 has been shortened can be confirmed by observing the graduations 36 (see FIGS. 2 and 3) provided on the outer circumferential surface of the sheath body 18.

As has been described above, the introducer sheath 14 ensures that after it is employed as an outer tube of the placement device 10 used for placement of the blood vessel treatment instrument (stent graft 12), the overall length of the sheath body 18 can be shortened. Therefore, a treatment device shorter than the overall length of the original introducer sheath 14 can be inserted into a blood vessel by utilizing the introducer sheath 14 shortened in effective length. In addition, it is unnecessary to employ a treatment device that is longer than needed.

According to the introducer sheath 14, it is possible, by taking up the terminal pieces 28a and 28b of the sheath body 18 by the plurality of take-up shafts 30 and 32, to draw the sheath body 18 into the hub 20 and simultaneously form cuts in the sheath body 18 by the cutting section 28. Therefore, by rotating the take-up shafts 30 and 32, take-up of the sheath body 18 and formation of the slits can be carried out concurrently. Accordingly, the effective length of the sheath body 18 can be easily shortened by a single operation (rotating operation).

In addition, in the introducer sheath 14, a proximal portion of the sheath body 18 is torn up into two by the cutting section 28, and the terminal ends thus torn apart are taken up by the two take-up shafts 30 and 32 spaced from each other in the direction of splitting of the sheath body 18. Therefore, the sheath body 18 can be smoothly taken up by a small number of component parts.

Further, in the introducer sheath 14, the sharp cutting blades 72 and 74 are used as the cutting section 28, whereby the cutting resistance at the time of drawing the sheath body 18 into the hub 20 can be reduced. This makes it possible to reduce the torque required for taking up the terminal pieces 28a and 28b of the sheath body 18, and to perform the taking-up (winding) operation more easily.

Furthermore, in the introducer sheath 14, the action of the interlocking mechanism 106 ensures that all the take-up shafts 30 and 32 can be simultaneously rotated by only rotationally operating either one of the operating wheels 100 and 102. Consequently the terminal pieces 28a and 28b torn apart can be taken up in a well-balanced manner. Accordingly, the sheath body 18 can be smoothly drawn into the inside of the hub body 31, and the taking-up operation can be carried out more easily.

In the introducer sheath 14, each part is sealed liquid-tight by the seal members 54, 56, 58 and 60. Therefore, even when blood flows into the hub 20 through the sheath body 18, the blood is prevented from leaking to the exterior of the hub 20.

FIG. 7 is a transverse sectional view of an introducer sheath 14a according to a modification. Now, the introducer sheath 14a according to the modification will be described below. In the following, description will be made to center on differences of the introducer sheath 14a from the above-described introducer sheath 14, and descriptions of the same items as above will be omitted.

The introducer sheath 14a shown in FIG. 7 has rotation restraining mechanisms 104 and 105 for inhibiting the take-up shafts 30 and 32 from rotating in an unwinding direction. In the configuration shown, the rotation restraining mechanisms 104 and 105 are composed of one-way clutches 104A and 105A. Of the one-way clutches 104A and 105A, outer circumferential portions are fixed to the trunk member 38 whereas inner circumferential portions are fixed to the take-up shafts 30 and 32.

Incidentally, in the case of this embodiment, the take-up shafts 30 and 32 are interlocked with each other through gears 107 and 108. Therefore, only one of the one-way clutches 104A and 105A may be provided.

In the introducer sheath 14a configured as above-mentioned, the take-up shafts 30 and 32 are inhibited by the rotation restraining mechanisms 104 and 105 from rotating in the unwinding direction. This ensures that the terminal pieces 28a and 28b of the sheath body 18 which have once been taken up would not be unwound, so that the sheath body 18 is inhibited from moving in the extending direction relative to the hub 20. Therefore, the slits formed by the cutting section 28 would not be exposed to the outside of the hub 20. Accordingly, blood can be prevented from leaking to the exterior of the hub 20 through the slits.

The configuration of the rotation restraining mechanisms 104 and 105 is not restricted to the one-way clutches 104A and 105A. The configuration may be a ratchet mechanism having an engagement section for engagement with at least one of the gears 107 and 108. The ratchet mechanism in this case is so configured that the engagement section is elastically displaced to ride over teeth of the gear(s) 107 and 108 at the time of rotation in the winding direction of the take-up shafts 30 and 32, whereas the engagement section does not ride over the teeth of the gear(s) 107 and 108 at the time of rotation in the reverse direction to the winding direction. This configuration inhibits the take-up shafts 30 and 32 from reverse rotation.

In the introducer sheaths 14 and 14a described above, the number of the portions in which the slits are formed by the cutting section 28 is not limited to two but may be three or more. In other words, the proximal portion of the sheath body 18 may be taken up after being torn up into three or more. Where the slits are thus formed in three or more portions, the number of the terminal pieces formed from the sheath body 18 is the same as the number of the slits. In this case, a configuration may be adopted wherein two take-up shafts 30 and 32 are provided in the same manner as above, and two of the plural (three or more) terminal pieces are taken up by the take-up shafts 30 and 32. Alternatively, a plurality of take-up shafts corresponding respectively to the terminal pieces formed in the same number as the number of the slits may be provided. In this case, the axes (rotational center lines) of the take-up shafts may not necessarily be parallel to one another.

FIG. 8 is a partly omitted perspective view of an introducer sheath 14b according to another modification. While the introducer sheath 14b will be described below, description will be made to center on differences of the introducer sheath 14b from the above-described introducer sheath 14, and descriptions of the same items as above will be omitted.

In the introducer sheath 14b shown in FIG. 8, a cutting section 120 for forming a plurality of slits in a sheath body 18 along the axial direction is composed of one or a plurality of linear members (in the example shown, two linear members 122 and 124). The linear members 122 and 124 may be, for example, wires, strings or the like.

In a hub body 31 in the example shown, two linear (filamentous) members 122 and 124 are disposed substantially in parallel so that plural (four) slits are formed in the sheath body 18 on the side of the distal end of the hub body 31 relative to take-up shafts. Therefore, a proximal portion of the sheath body 18 is split into four terminal pieces 126a to 126d.

In the example shown, the two linear members 122 and 124 are fixed to a distal-side wall surface 128 of a trunk member 38 by appropriate means, and is so disposed as to traverse a distal-side opening 46 (see FIG. 4). In place of the layout configuration shown, a configuration may be adopted in which the two linear members 122 and 124 are embedded in a distal component section 50 so as to traverse the inside of the distal-side opening 46. The linear members 122 and 124 are stretched with an appropriate tension exerted thereon so that the slits can be effectively formed in the sheath body 18 when the sheath body 18 is drawn into the hub 20. In order to reduce the cutting resistance at the time of forming the slits in the sheath body 18, it is preferable for the linear members 122 and 124 to be sufficiently small in diameter within a range in which strength can be secured.

In the condition where an inner tube body 22 is inserted in the sheath body 18, a part between both ends of each of the linear members 122 and 124 is interposed between the sheath body 18 and the inner tube body 22. The two linear members 122 and 124 are disposed at positions spaced from each other in the axial direction of the take-up shafts 30 and 32, in such a manner as to extend in a direction substantially orthogonal to the axial direction of the take-up shafts 30 and 32. Therefore, as shown in FIG. 8, two of the four terminal pieces 126a to 126b are split to both sides with respect to the spacing direction of the take-up shafts 30 and 32, whereas the other two are split to both sides with respect to the axial direction of the take-up shafts 30 and 32. The terminal pieces 126a and 126c are held by (fixed to) the take-up shafts 30 and 32, respectively. The remaining terminal pieces 126b and 126d are left in the state of free ends, without being fixed to any other member or element in a hollow section 44.

In order to shorten the effective length of the sheath body 18 in the introducer sheath 14b, it suffices to rotate an operating section 34, like in the introducer sheath 14 shown in FIG. 2, etc. Specifically, when operating wheels 100 and 102 are rotated, the take-up shafts 30 and 32 connected to the operating wheels 100 and 102 are rotated and the terminal pieces 126a and 126c are taken up by the take-up shafts 30 and 32, simultaneously when the slits are formed in the sheath body 18 by the linear members 122 and 124. As a result, the sheath body 18 is drawn into the hub 20, and the effective length thereof is shortened.

While the terminal pieces 126a and 126c are taken up by the take-up shafts 30 and 32 as above-mentioned, the other terminal pieces 126b and 126d are not taken up. However, the terminal pieces 126b and 126d are flexible and can be easily deformed when an external force acts thereon; therefore, the terminal pieces 126b and 126d are deformed inside the hollow section 44 upon making contact with an inner wall constituting the hollow section 44, during when the terminal pieces 126a and 126c are taken up by the take-up shafts 30 and 32. Accordingly, take-up of the terminal pieces 126a and 126c by the take-up shafts 30 and 32 would not be hampered.

In order to respectively take up the terminal pieces 126b and 126d, the hub body 31 may be further provided with other two take-up shafts. In this case, the take-up shafts for taking up the terminal pieces 126b and 126d are provided at positions which are on the side of the distal end or proximal end of the hub body 31 in relation to the take-up shafts 30 and 32 and which are on opposite sides with reference to the axis of the hub 20 (the axis of the distal-side opening 46 and the proximal-side opening 48 (see FIG. 4)). In addition, the take-up shafts for taking up the terminal pieces 126b and 126d are rotatably provided on the trunk member 38, in such a manner that their axial direction is orthogonal to the axial direction of the take-up shafts 30 and 32.

In FIG. 8, a configuration wherein the two linear members 122 and 124 are provided has been shown as a configuration example of the cutting section 120. The cutting section 120 may have another configuration wherein a single linear member is disposed in a bent or crooked form such as to form four slits in the sheath body 18. Specifically, a configuration may be adopted wherein a part of a single linear member is disposed in the same manner as the linear member 122, and another part of the single linear member is disposed in the same manner as the linear member 124. This configuration ensures that four slits are formed in the sheath body 18 so that the sheath body 18 is split into the terminal pieces 126a to 126d.

Incidentally, the introducer sheath 14b may be provided with the rotation restraining mechanisms 104 and 105 shown in FIG. 7.

While the present invention has been described above by showing some preferred embodiments thereof, the invention is not to be restricted to the above embodiments. Naturally, various alterations are possible within the scope of the gist of the invention.

## Claims

1. An introducer sheath (14, 14a, 14b) into which a long shaft is inserted, comprising:
a flexible tube-shaped sheath body (18); and
a hub (20) into which a proximal portion of the sheath body (18) is inserted,
wherein the hub (20) takes up a proximal portion of the sheath body (18) into the hub (20) while tearing the proximal portion of the sheath body (18), whereby the length of extension of the sheath body (18) from the hub (20) can be shortened.

2. The introducer sheath (14, 14a, 14b) according to claim 1,
wherein the hub (20) has:
a cutting section (28, 120) by which slits along an axial direction are formed in the sheath body (18); and
a plurality of take-up shafts (30, 32) for respectively taking up terminal pieces of the sheath body (18) torn by the slits.

3. The introducer sheath (14, 14a, 14b) according to claim 2,
wherein the cutting section (28, 120) forms the slits in portions of the sheath body (18) which are on opposite sides with respect to a circumferential direction, and
the plurality of take-up shafts (30, 32) are two take-up shafts (30, 32) disposed at positions spaced from each other along the direction of splitting of the sheath body (18) by the slits.

4. The introducer sheath (14, 14a) according to claim 2,
wherein the cutting section (28, 120) has a plurality of cutting blades (72, 74) by which the slits are formed in circumferential-directionally different portions of the sheath body (18).

5. The introducer sheath (14, 14a, 14b) according to claim 2,
wherein the hub (20) further has an interlocking mechanism (106) by which the plurality of take-up shafts (30, 32) are rotated in an interlocked manner.

6. The introducer sheath (14a) according to claim 2,
wherein the hub (20) has rotation restraining mechanisms (104, 105) for inhibiting the take-up shafts (30, 32) from rotating in an unwinding direction.

7. The introducer sheath (14, 14a, 14b) according to claim 2,
wherein the hub (20) has:
a hub body (31) provided with a hollow section in which the take-up shafts (30, 32) and the proximal portion of the sheath body (18) are housed; and
a rotational operating section (34) which is rotationally operated from outside of the hub body (31) to thereby rotate the take-up shafts (30, 32),
the hub body (31) being configured to be liquid-tight so that a liquid flowing into the inside of the hub (20) through the sheath body (18) does not leak to the exterior.

8. A blood vessel treatment instrument placement device (10) by which a blood vessel treatment instrument (12) having a self-expanding function is fed to and placed indwelling in a desired treatment site in a blood vessel,
wherein the placement device (10) includes an introducer sheath (14, 14a, 14b) having a sheath body (18) for housing the blood vessel treatment instrument (12) on an inner circumference of a distal portion thereof, and
an inner tube (16) slidably inserted inside the sheath body (18);
the introducer sheath (14, 14a, 14b) has
the sheath body (18) which is flexible and tube-like in shape, and
a hub (20) in which a proximal portion of the sheath body (18) is inserted; and
the hub (20) takes up a proximal portion of the sheath body (18) into the hub (20) while tearing the proximal portion of the sheath body (18), whereby the length of extension of the sheath body (18) from the hub (20) can be shortened.

9. A method for shortening an introducer sheath (14, 14a, 14b) in which a long shaft is inserted, the method comprising:
tearing a proximal portion of a sheath body (18);
drawing the sheath body (18) into the hub (20) in which the proximal portion of the sheath body (18) is inserted; and
taking up terminal pieces of the sheath body (18) having been torn.
